# EUROPEAN PATENT SPECIFICATION

(11) **EP 2 322 161 B1**
(45) Date of publication and mention of the grant of the patent: **31.12.2014**
(21) Application number: 10191089.1
(22) Date of filing: 12.11.2010
(51) Int. Cl.: A61K 31/133, A61K 31/155, A61K 31/198, A61K 45/06, A61K 9/00, A61Q 11/00, A61P 31/02, A61P 29/00

(54) **Composition for preventing and treating odontostomatological pathologies of animals**
Zusammensetzung zur Prävention und Behandlung von odontostomatologischen Pathologienbei Tieren
Composition pour prévenir et traiter les pathologies odontostomatologiques dans les animaux

(30) Priority: 16.11.2009 IT MI20092010
(43) Date of publication of application: 18.05.2011
(73) Proprietor: I.C.F. S.r.l., 26020 Palazzo Pignano (CR) (IT)
(72) Inventor: Falanga, Gennaro, 26040 Gerre de Caprioli (CR) (IT)
(74) Representative: Finetti, Claudia

(56) References cited:
- EP-A1- 1 595 537
- WO-A1-02/24201
- WO-A1-2005/079747
- WO-A1-2009/104963
- WO-A2-2004/096140
- US-A1- 2005 064 019
- ZA-A- 9 900 349
- HARPER W E S: "Simple additives to increase the activity of chlorhexidine digluconate against urinary pathogens", PARAPLEGIA 1983 GB, vol. 21, no. 2, 1983, pages 86-93, XP002593253, ISSN: 0031-1758
- DATABASE WPI Section Ch, Week 200174 5 June 2001 (2001-06-05) Thomson Scientific, London, GB; Class D21, AN 2001-646314 XP002593798, & KR 2001 045 670 A (INTERCOSM BIOTECH LAB INC) 5 June 2001 (2001-06-05)
- BIBEL DEBRA JAN ET AL: "Inhibition of microbial adherence by sphinganine", CANADIAN JOURNAL OF MICROBIOLOGY, vol. 38, no. 9, 1992, pages 983-985, XP002593255, ISSN: 0008-4166

## Description

The present invention relates to a composition for the prevention and treatment of odontostomatological pathologies of animals and the relative uses.

In particular, the present invention relates to a composition for the prevention of the formation of plaque and tartar in animals and for the treatment of odontostomatological pathologies of animals of an infective and/or traumatic nature.

In the state of the art, the treatment of odontostomatological pathologies of animals, such as for example the treatment of dental extractions, ulcerative stomatitis, granulomas, periodontitis, infections of the oral cavity, plaque, tartar, traumatic lesions at the level of the oral cavity, is based on systemic therapies which use pharmaceutical products of the antibiotic type based on chlorhexidine or derivatives thereof.

Chlorhexidine is an antibacterial compound with a wide range, used in the preparation of numerous pharmaceutical products, in which its proven bacteriostatic, bactericide, antimycotic and antiviral action is exploited together with its capacity of preventing and slowing down the formation of tartar and plaque. Chlorhexidine is also used for the preparation of products destined for human beings, such as, for example, mouthwashes, toothpastes, gingival pastes, etc..

In the veterinary field, the use of compositions based on chlorhexidine known in the state of the art for the treatment of odontostomatological pathologies of animals has various drawbacks, mainly associated with the high dosage which must be administered to animals in order to obtain significant benefits.

A first disadvantage which derives from the use of chlorhexidine at a high dosage, for example, is linked to the outbreak of side-effects, such as for example yellow colouring (reversible) of the tooth enamel in animals with odontostomatological pathologies of a bacterial, mycotic, viral origin, treated twice a day, for periods longer than 7 consecutive days, with a concentration equal to or higher than 0.12%. The outbreak of these side-effects can make it necessary to interrupt the treatment before the desired results have been reached.

A second disadvantage associated with the use of the compositions based on chlorhexidine of the state of the art is linked to the fact that although these compositions exert the antimicrobial action described above, they are not always sufficiently effective with respect to possible inflammatory states. In the presence of pathologies accompanied by inflammatory states, it is consequently often necessary to use further pharmaceutical compositions having a specific anti-inflammatory activity.

An objective of the present invention is to overcome the disadvantages of the known art.

In particular, one of the main objectives of the present invention consists in providing a composition for veterinary use capable of effectively treating odontostomatological pathologies of animals using low dosages of chlorhexidine.

Another objective of the present invention consists in providing a composition for preventing the formation of plaque and tartar in the veterinary field.

A further objective of the present invention consists in providing a composition for the treatment of odontostomatological pathologies in the veterinary field, capable of acting as antimicrobial product and, at the same time, as anti-inflammatory product.

A first object of the present invention relates to a composition for the prevention and treatment of odontostomatological pathologies of animals comprising
(a) from 0.002 to 0.5% by weight, with respect to the total weight of the composition, of chlorhexidine and/or a derivative thereof selected from the group consisting of chlorhexidine digluconate, chlorhexidine dichloride, chlorhexidine diacetate and/or mixtures thereof,
(b) from 0.0001 to 5% by weight, with respect to the total weight of the composition, of phytosphingosine and/or a derivative thereof selected from N-substituted phytosphingosine compounds, preferably N-loctyloyl and N-salicyloyl, and phytosphingosine salts with hydrophilic acids, preferably with lactic acid, glycolic acid, succinic acid, hydrochloric acid, nitric acid and phosphoric acid, and
(c) from 0.002 to 48% by weight, with respect to the total weight of the composition, of a Tris-EDTA buffer solution having a pH that can vary from 7.4 to 8.6
as active ingredients, in combination with adjuvants and/or excipients acceptable in the veterinary field.

A further object of the present invention relates to possible specific uses of the above composition described hereunder.

The Applicant has surprisingly found that by combining chlorhexidine and/or its derivatives, phytosphingosine and/or its derivatives and a buffer solution of Tris-EDTA, in suitable weight ratios, a composition can be obtained for veterinary use, having a high antimicrobial and, at the same time, anti-inflammatory effectiveness.

The use of the composition object of the present invention in the veterinary field has an effectiveness comparable to or higher than that shown by the chlorhexidine-based compositions used in the state of the art for the treatment of odontostomatological pathologies of animals, and also for preventing the formation of plaque and tartar in the veterinary field, considerably reducing the outbreak of side-effects associated with this compound.

Thanks to the synergic effect deriving from the combination of its components, in fact, the composition, object of the present invention, has a high efficacy also at low dosages of chlorhexidine.

The composition, object of the present invention, is characterized by the capacity of contemporaneously exerting an action of both the antimicrobial and anti-inflammatory type.

For preparing the composition for veterinary use, object of the present invention, it is possible to use chlorhexidine as such, i.e. chlorhexidine in free form, or one of its derivatives. The derivative of chlorhexidine is preferably selected from the group consisting of chlorhexidine digluconate, chlorhexidine dichloride, chlorhexidine diacetate and/or mixtures thereof.

Chlorhexidine and its derivatives can be synthesized according to method known in the state of the art. They are commercially available, generally in the form of aqueous solutions with different titres.

In the composition for veterinary use, object of the present invention, the chlorhexidine and/or its derivatives are present in an overall amount ranging from 0.002 to 0.5% by weight with respect to the total weight of the composition, preferably from 0.01 to 0.2%, more preferably from 0.04 to 0.1%.

Phytosphingosine is a compound of a natural origin having the molecular formula C₁₈H₃₉NO₃ and belonging to the group of sphingoid bases.

For the purposes of the present invention, the phytosphingosine can be used as such, i.e. phytosphingosine in free form or in the form of one of its N-substituted compounds (for example N-loctyloyl and N-salicyloyl), or one of its salts obtained by reaction of phytosphingosine with hydrophilic acids, (for example: lactic acid, glycolic acid, succinic acid, hydrochloric acid, nitric acid and phosphoric acid).

The anti-inflammatory activity of phytosphingosine and its derivatives is linked to the capacity of these compounds of inhibiting the pro-inflammatory cytokines released by mastocytes.

According to the most reliable theories in the state of the art, mastocytes activated by super-maximum immunogenic stimuli are mainly responsible for local hyper-reactivity of the cells. The mastocytes release cytokines (low-molecular-weight proteins) which act locally as chemical messengers binding themselves to cell receptors, which, once activated, transmit a pro-inflammatory signal inside the cells.

Phytosphingosine and its derivatives are also capable of significantly limiting the growth of Gram-positive and Gram-negative bacteria, preventing their adherence to the oral mucosa.

Phytosphingosine and its derivatives are compounds of a natural origin which can also be obtained synthetically. The are commercially available.

In the composition for veterinary use, object of the present invention, the phytosphingosine and its derivatives are present in an overall amount ranging from 0.0001 to 5% by weight with respect to the total weight of the composition, preferably from 0.001 to 1%, even more preferably from 0.01 to 0.5%.

A third essential component of the composition, object of the present invention, is a buffer solution of the Tris-EDTA type. It ha been surprisingly found that the presence of this type of buffer solution reinforces the action of chlorhexidine and its derivatives, thus allowing the use of these compounds, with the same treatment efficacy, in lower quantities with respect to compositions based on chlorhexidine and its derivatives known in the state of the art and used for the same veterinary purposes.

For the purposes of the present invention, the term Tris-EDTA indicates a buffer solution comprising tris(hydroxymethyl)aminomethane (Tris), ethylenediaminotetraacetic acid (EDTA) and/or a derivative of EDTA.

The composition preferably comprises a derivative of EDTA, in particular disodium salt dihydrate (Na₂EDTA•2H₂O) .

Buffer solutions of the Tris-EDTA type which can be used for the purposes of the present invention are commercially available and have a pH ranging from 7.4 to 8.6.

In the composition for veterinary use, object of the present invention, the Tris-EDTA buffer solution is present in an amount ranging from 0.002 to 48% by weight with respect to the total weight of the composition, preferably from 0.02 to 35%, more preferably from 0.1 to 15%.

In the composition, object of the present invention, the Tris-EDTA buffer solution preferably comprises:
- the Tris compound in an amount which is such that its percentage weight, referring to the overall weight of the composition, is variable from 0.001% to 30%, more preferably from 0.01% to 25%, even more preferably from 0.05% to 10%;
- the EDTA compound and/or a derivative thereof in an amount which is such that the percentage weight of the EDTA compound and/or its derivative, referring to the overall weight of the composition, is variable from 0.001% to 18%, more preferably from 0.01% to 10%, even more preferably from 0.05% to 5%.

In a preferred embodiment of the invention, the composition for veterinary use also comprises one or more fatty acids of the omega-6 type. The omega-6 fatty acids used preferably comprise linoleic acid.

It has been found, in fact, that omega-6 fatty acids have an additional anti-inflammatory action which, unlike the corticosteroids generally used in compositions for odontostomatological use in the veterinary field known in the art, has no side-effects.

In the composition for veterinary use, object of the present invention, the omega-6 fatty acids are present in an overall amount ranging from 0 to 50% by weight with respect to the total weight of the composition, preferably from 0 to 25%, more preferably from 0 to 10%.

In order to be used in a veterinary context, the composition, object of the present invention, also comprises adjuvants and/or excipients acceptable for this purpose. The adjuvants and/or excipients which can be used are known to experts in the field.

The composition, object of the present invention, preferably comprises adjuvants and/or excipients of the following type: thickening agents, ethoxylated coconut fatty acids, sorbitol, emulsifying agents, glycerine and preservatives.

To give a more pleasant taste to the composition, object of the present invention and facilitate its administration to animals, it is preferable to also add one or more aromas, preferably meat, fish aromas, vegetable extracts or fruit, to the composition.

In a preferred embodiment, the composition, object of the present invention, is formulated in a form suitable for oral administration.

The composition is typically formulated in the form of gels, sprays, mouthwashes, foaming bases, lotions, emulsions and spot-on products. The composition is more preferably formulated in the form of gels, sprays, mouthwashes or emulsions.

Considering the fact that chlorhexidine and its derivatives also exert an action for preventing and reducing the formation of bacterial plaque on dental surfaces, the composition, object of the present invention, can also have a cosmetic use for preventing the formation of plaque and slowing down the formation of tartar, in this way also reducing halitosis. In this case, the composition is formulated with cosmetically acceptable adjuvants and/or excipients.

The composition, object of the present invention, is prepared according to the methods and with the equipment known to experts in the field.

The various ingredients are typically mixed with the Tris-EDTA buffer solution in the weight ratios defined above. The composition, moreover, also contains demineralised water in a variable amount in relation to the formulation to be prepared.

The amount of water to be added to the composition can be easily determined by an expert in the field.

A further object of the present invention is represented by the possible specific uses of the above composition described above.

An additional object of the present invention relates to the use of the composition described above as an anti-inflammatory agent at an odontostomatological level, in particular, for treating inflammatory states of the vestibule of the mouth and/or gums of animals.

A further object of the present invention relates to the use of the composition described above as an antimicrobial agent at an odontostomatological level.

Yet another object of the present invention relates to the use of the composition described above as an anti-plaque and anti-tartar agent at an odontostomatological level.

The above uses refer to the treatment of odontostomatological pathologies of all animal species having dentures, preferably dogs, cats and reptiles.

The dosage of the composition, object of the present invention, is in relation to the body weight of the animal, the odontostomatological pathology to be treated and the type of formulation of the composition.

In the case of a formulation in the form of a gel destined for the treatment of a stomatitis in dogs, for example, the composition, object of the present invention, is applied to the vestibule of the mouth and on the surface of the gums of the animal preferably once or twice a day, with the quantity necessary for covering the whole area of interest. The application of the gel is preferably effected between meals.

The treatment with the composition, object of the present invention, according to this dosage can be repeated until the complete healing of the pathology.

The composition, object of the present invention, can also be used for maintenance therapies in chronic or recurring forms of the pathologies afflicting animals at an odontostomatological level, or for preventive treatment of the same pathologies.

The following embodiment examples are provided for purely illustrative purposes of the present invention and should not be considered as limiting the protection scope defined by the enclosed claims.

### EXAMPLE 1

According to the present invention, 100 g of a composition for veterinary use were prepared in the form of a gel by mixing:
- 0.06 g of phytosphingosine hydrochloride,
- 0.3 g of a 20% solution by weight of chlorhexidine digluconate,
- 1.0 g of disodium dihydrogen ethylenediaminotetracetic acid,
- 3.8 g of tris(hydroxymethyl)aminomethane,
- 2.25 g of omega 6 fatty acids,
- 0.4 g of meat flavouring,
- 1.05 g of hydroxyethyl cellulose,
- 0.5 g of a 30% solution by weight of 2-pyrrolidone, 1-ethenyl-homopolymer,
- 0.2 g of ethoxylated coconut fatty acids,
- 7 g of a 70% solution by weight of sorbitol,
- 0.1 g of emulsifier,
- 30 g of glycerine,
- 0.1 g of preservative,
- Demineralised water up to a total weight of the composition equal to 100 g.

The composition was used in the treatment of a bacterial ulcerative stomatitis in a dog twice a day for 10 days, applying the product until all the gums were covered.

The use of the composition according to the present invention allowed the animal to be healed from bacterial ulcerative stomatitis, without the appearance of side-effects typically encountered in therapies with compositions based on chlorhexidine known in the state of the art.

## Claims

1. Composition for use in the prevention and treatment of odontostomatological pathologies of animals comprising
a) from 0.002 to 0.5% by weight, with respect to the total weight of the composition, of chlorhexidine and/or a derivative thereof selected from the group consisting of chlorhexidine digluconate, chlorhexidine dichloride, chlorhexidine diacetate and/or mixtures thereof,
b) from 0.0001 to 5% by weight with respect to the total weight of the composition, of phytosphingosine and/or a derivative thereof selected from N-substituted phytosphingosine compounds, preferably N-loctyloyl and N-salicyloyl, and phytosphingosine salts with hydrophilic acids, preferably with lactic acid, glycolic acid, succinic acid, hydrochloric acid, nitric acid and phosphoric acid, and
c) from 0.002 to 48% by weight with respect to the total weight of the composition, of a Tris-EDTA buffer solution having a pH that can vary from 7.4 to 8.6
as active ingredients, in combination with adjuvants and/or excipients that are acceptable in the veterinary field.

2. Composition for use according to the previous claim, also comprising one or more omega-6 type fatty acids in a variable amount between 0 and 50% by weight, with respect to the total weight of the composition, preferably between 0 and 25%, more preferably between 0 and 10%.

3. Composition for use according to any one of the previous claims, wherein the chlorhexidine and/or its derivatives are present overall in a variable amount between 0.01 and 0.2%, preferably between 0.04 and 0.1%.

4. Composition for use according to any one of the previous claims, wherein the phytosphingosine and/or its derivatives are present overall in a variable amount between 0.001 and 1%, even more preferably between 0.01 and 0.5%.

5. Composition for use according to any one of the previous claims, wherein the phytosphingosine is present as phytosphingosine hydrochloride.

6. Composition for use according to any one of the previous claims, wherein the Tris-EDTA buffer solution is present in a variable amount between 0.02 and 35% by weight, with respect to the total weight of the composition, preferably between 0.1 and 15% by weight.

7. Composition for use according to any one of the previous claims, wherein the Tris-EDTA buffer solution comprises:
- the Tris compound in an amount such that its percentage weight, referring to the overall weight of the composition, is variable from 0.001% to 30%, more preferably from 0.01% to 25%, even more preferably from 0.05% to 10%;
- the EDTA compound and/or its derivative disodium dihydrogen ethylenediaminotetracetic acid in an amount such that the percentage weight of said EDTA compound and/or its derivative disodium dihydrogen ethylenediaminotetracetic acid, referring to the overall weight of the composition, is variable from 0.001% to 18%, more preferably from 0.01% to 10%, even more preferably from 0.05% to 5%.

8. Composition for use according to any one of the previous claims formulated in a form suitable for oral administration selected from the group consisting of gel, spray, mouthwash, foaming base, lotion, emulsion and spot-on.

9. Composition for use according to claim 1 having the following percentage composition by weight:
- 0.06 g of phytosphingosine hydrochloride,
- 0.3 g of a 20% solution by weight of chlorhexidine, digluconate,
- 1.0 g of disodium dihydrogen ethylenediaminotetracetic acid,
- 3.8 g of tris(hydroxymethyl)aminomethane,
- 2.25 g of omega 6 fatty acids,
- 0.4 g of meat flavouring,
- 1.05 g of hydroxyethyl cellulose,
- 0.5 g of a 30% solution by weight of 2-pyrrolidone, 1-ethenyl-homopolymer,
- 0.2 g of ethoxylated coconut fatty acids,
- 7 g of a 70% solution by weight of sorbitol,
- 0.1 g of emulsifier,
- 30 g of.glycerine,
- 0.1 g of preservative,
- Demineralised water up to a total weight of the composition equal to 100 g.

10. Composition according to any one of the previous claims as anti-inflammatory and anti-microbial drug for use in the treatment of odontostomatological pathologies of animals.

11. Composition according to any one of the previous claims from 1 to 9 as anti-plaque and anti-tartar agent for use in the treatment of odontostomatological pathologies of animals.

12. Cosmetic use of the composition according to any one of the previous claims from 1 to 9 for reducing halitosis on the teeth surfaces of animals.

13. Use of the composition according to any one of the previous claims from 1 to 9 in the manufacture of a medicament for preventing the formation of bacterial plaque and/or tartar, on the teeth surfaces of animals.

## Patentansprüche

1. Zusammensetzung zur Prävention und Behandlung von odontostomatologischen Pathologien bei Tieren, umfassend
a) zwischen 0,002 und 0,5 Gew.-% Chlorhexidin und/oder ein Derivat hiervon - bezogen auf das Gesamtgewicht der Zusammensetzung - ausgewählt aus der Gruppe bestehend aus Chlorhexidindiglocunat, Chlorhexidindichlorid, Chlorhexidindiacetat und/oder Gemischen hiervon,
b) zwischen 0,0001 und 5 Gew.% Phytosphingosin und/oder ein Derivat hiervon - bezogen auf das Gesamtgewicht der Zusammensetzung - ausgewählt aus N-substituierten Phytosphingosin-Verbindungen, bevorzugt N-Loctyloyl und N-Salicyloyl, und Phytosphingosinsalzen mit hydrophilen Säuren, bevorzugt mit Milchsäure, Glykolsäure, Bernsteinsäure, Salzsäure, Salpetersäure und Phosphorsäure, und
c) zwischen 0,002 und 48 Gew.-% - bezogen auf das Gesamtgewicht der Zusammensetzung - einer Tris-EDTA-Pufferlösung mit einem pH-Wert, der zwischen 7,4 und 8,6 variabel ist,
als Wirkstoffe in Verbindung mit Hilfsstoffen und/oder Trägerstoffen, die auf veterinärmedizinischem Gebiet zulässig sind.

2. Zusammensetzung zur Verwendung nach dem vorstehenden Anspruch, ebenfalls umfassend eine oder mehrere Omega-6-Fettsäuren in einer - bezogen auf das Gesamtgewicht der Zusammensetzung - zwischen 0 und 50 Gew.-%, bevorzugt zwischen 0 und 25 Gew.-%, mehr bevorzugt zwischen 0 und 10 Gew.-% variablen Menge.

3. Zusammensetzung zur Verwendung nach einem beliebigen der vorstehenden Ansprüche, wobei Chlorhexidin und/oder dessen Derivate insgesamt in einer zwischen 0,01 und 0,2 Gew.-%, bevorzugt zwischen 0,04 und 0,1 Gew.-% variablen Menge vorliegt.

4. Zusammensetzung zur Verwendung nach einem beliebigen der vorstehenden Ansprüche, wobei Phytosphingosin und/oder dessen Derivate insgesamt in einer zwischen 0,001 und 1 Gew.-%, und noch mehr bevorzugt zwischen 0,01 und 0,5 Gew.-% variablen Menge vorliegt.

5. Zusammensetzung zur Verwendung nach einem beliebigen der vorstehenden Ansprüche, wobei Phytoshingosin als Phytosphingosinhydrochlorid vorliegt.

6. Zusammensetzung zur Verwendung nach einem beliebigen der vorstehenden Ansprüche, wobei die Tris-EDTA-Pufferlösung in einer - bezogen auf das Gesamtgewicht der Zusammensetzung - zwischen 0,02 und 35 Gew.-%, bevorzugt zwischen 0,1 und 15 Gew.-% variablen Menge vorliegt.

7. Zusammensetzung zur Verwendung nach einem beliebigen der vorstehenden Ansprüche, wobei die Tris-EDTA-Pufferlösung Folgendes umfasst:
- die Tris-Verbindung in einer solchen Menge, dass ihr Gewichtsprozentsatz - bezogen auf das Gesamtgewicht der Zusammensetzung - zwischen 0,001 und 30 Gew.-%, mehr bevorzugt zwischen 0,01 und 25 Gew.-% und noch mehr bevorzugt zwischen 0,05 und 10 Gew.-% variabel ist;
- die EDTA-Verbindung und/oder ihr Derivat Dinatriumdihydrogen-Ethylendiamintetraessigsäure in einer solchen Menge, dass der Gewichtsprozentsatz der EDTA-Verbindung und/oder ihres Derivats Dinatriumdihydrogen-Ethylendiamintetraessigsäure - bezogen auf das Gesamtgewicht der Zusammensetzung - zwischen 0,001 und 18 Gew.-%, mehr bevorzugt zwischen 0,01 und 10 Gew.-% und noch mehr bevorzugt zwischen 0,05 und 5 Gew.-% variabel ist.

8. Zusammensetzung zur Verwendung nach einem beliebigen der vorstehenden Ansprüche, formuliert in einer für die orale Verabreichung angemessenen Form, ausgewählt aus der Gruppe bestehend aus Gel, Spray, Mundspülung, Schaumbasis, Lotion, Emulsion und Auftupfpräparat.

9. Zusammensetzung zur Verwendung nach Anspruch 1, die folgende Gewichtsprozentsätze aufweist:
- 0,06 g Phytosphingosinhydrochlorid,
- 0,3 g einer 20 gew.-% igen Chlorhexidindigluconat-Lösung,
- 1,0 g Dinatriumdihydrogen-Ethylendiamintetraessigsäure,
- 3,8 g Tris(hydroxymethyl)-aminomethan,
- 2,25 g Omega-6-Fettsäuren,
- 0,4 g Fleischaroma,
- 1,05 g Hydroxyethylzellulose,
- 0,5 g einer 30 gew.-%igen Lösung von 1-Ethenyl-2-Pyrrolidon-Homopolymer,
- 0,2 g ethoxylierte Kokosfettsäuren,
- 7 g einer 70 gew.-%igen Sorbitollösung,
- 0,1 g Emulgator,
- 30 g Glycerin,
- 0,1 g Konservierungsstoff
- entmineralisiertes Wasser bis zu einem Gesamtgewicht der Zusammensetzung von 100 g.

10. Zusammensetzung nach einem beliebigen der vorstehenden Ansprüche als entzündungshemmendes und antimikrobielles Arzneimittel zur Verwendung in der Behandlung von odontostomatologischen Pathologien bei Tieren.

11. Zusammensetzung nach einem beliebigen der vorstehenden Ansprüche von 1 bis 9 als Zahnbelag hemmender und Zahnstein hemmender Wirkstoff zur Verwendung in der Behandlung von odontostomatologischen Pathologien bei Tieren.

12. Kosmetische Verwendung der Zusammensetzung nach einem beliebigen der Ansprüche 1 bis 9 zur Verminderung von Halitosis auf den Zahnoberflächen von Tieren.

13. Verwendung der Zusammensetzung nach einem beliebigen der vorstehenden Ansprüche 1 bis 9 bei der Herstellung eines Medikaments zur Verhinderung der Bildung von bakteriellen Belägen und/oder Zahnstein auf den Zahnoberflächen von Tieren.

## Revendications

1. Composition pour l'utilisation dans la prévention et le traitement de pathologies odonto-stomatologiques chez les animaux comprenant
a) de 0,002 à 0,5% en poids, par rapport au poids total de la composition, de chlorhexidine et/ou un dérivé de celle-ci, sélectionné à partir du groupe comprenant le digluconate de chlorhexidine, le dichlorure de chlorhexidine, le diacétate de chlorhexidine et/ou des mélanges de ceux-ci,
b) de 0,0001 à 5% en poids par rapport au poids total de la composition, de phytosphingosine et/ou un dérivé de celle-ci, sélectionné à partir de composés de phytosphingosine N-substituée, de préférence N-loctyloyl et N-salicyloyl, et des sels de phytosphingosine avec des acides hydrophiles, de préférence avec l'acide lactique, l'acide glycolique, l'acide succinique, l'acide chlorhydrique, l'acide nitrique et l'acide phosphorique, et
c) de 0,002 à 48% en poids par rapport au poids total de la composition, d'une solution tampon Tris-EDTA ayant un pH qui peut varier de 7,4 à 8,6
comme ingrédients actifs, en combinaison avec des adjuvants et/ou des excipients qui sont admissibles dans le domaine vétérinaire.

2. Composition pour l'utilisation selon la revendication précédente, comprenant également un ou plusieurs acides gras de type oméga-6 dans une quantité variable entre 0 et 50% en poids, par rapport au poids total de la composition, de préférence entre 0 et 25%, de manière davantage préférée entre 0 et 10%.

3. Composition pour l'utilisation selon l'une quelconque des revendications précédentes, dans laquelle la chlorhexidine et/ou ses dérivés sont présents globalement dans une quantité variable entre 0,01 et 0,2%, de préférence entre 0,04 et 0,1%.

4. Composition pour l'utilisation selon l'une quelconque des revendications précédentes, dans laquelle la phytosphingosine et/ou ses dérivés sont présents globalement dans une quantité variable entre 0,001 et 1%, de manière encore plus préférée entre 0,01 et 0,5%.

5. Composition pour l'utilisation selon l'une quelconque des revendications précédentes, dans laquelle la phytosphingosine est présente en tant que chlorhydrate de phytosphingosine.

6. Composition pour l'utilisation selon l'une quelconque des revendications précédentes, dans laquelle la solution tampon Tris-EDTA est présente dans une quantité variable entre 0,02 et 35% en poids, par rapport au poids total de la composition, de préférence entre 0,1 et 15% en poids.

7. Composition pour l'utilisation selon l'une quelconque des revendications précédentes, dans laquelle la solution tampon Tris-EDTA comprend :
- le composé Tris dans une quantité telle que son pourcentage en poids, en se référant au poids total de la composition, soit variable de 0,001% à 30%, de manière davantage préférée de 0,01% à 25%, de manière encore plus préférée de 0,05% à 10%;
- le composé EDTA et/ou son dérivé l'acide éthylènediaminetétraacétique dihydrogénodisodique dans une quantité telle que le pourcentage en poids dudit composé EDTA et/ou son dérivé l'acide éthylènediaminetétraacétique dihydrogénodisodique, en se référant au poids total de la composition, soit variable de 0,001% à 18%, de manière davantage préférée de 0,01% à 10%, de manière encore plus préférée de 0,05% à 5%.

8. Composition pour l'utilisation selon l'une quelconque des revendications précédentes formulée sous une forme appropriée pour l'administration orale sélectionnée à partir du groupe comprenant un gel, un spray, un rince-bouche, une base moussante, une lotion, une émulsion et un spot-on.

9. Composition pour l'utilisation selon la revendication 1 ayant la composition en pourcentage en poids suivante :
- 0,06 g de chlorhydrate de phytosphingosine,
- 0,3 g of a 20% solution en poids de digluconate de chlorhexidine,
- 1,0 g d'acide éthylènediaminetétraacétique dihydrogénodisodique,
- 3,8 g de tris(hydroxyméthyl)aminométhane,
- 2,25 g d'acides gras oméga 6,
- 0,4 g d'arôme de viande,
- 1,05 g d'hydroxyéthylcellulose,
- 0,5 g d'une solution à 30% en poids de 2-pyrrolidone, 1-éthényl-homopolymère,
- 0,2 g d'acides gras de coco éthoxylés,
- 7 g d'une solution à 70% en poids de sorbitol,
- 0,1 g d'émulsifiant,
- 30 g de glycérine,
- 0,1 g d'agent de conservation,
- Eau déminéralisée jusqu'à un poids total de la composition égal à 100 g.

10. Composition selon l'une quelconque des revendications précédentes comme médicament anti-inflammatoire et antimicrobien pour l'utilisation dans le traitement de pathologies odonto-stomatologiques chez les animaux.

11. Composition selon l'une quelconque des revendications précédentes 1 à 9 comme agent anti-plaque et antitartre pour l'utilisation dans le traitement de pathologies odonto-stomatologiques chez les animaux.

12. Utilisation cosmétique de la composition selon l'une quelconque des revendications précédentes 1 à 9 pour la réduction de l'halitose sur les surfaces des dents d'animaux.

13. Utilisation de la composition selon l'une quelconque des revendications précédentes 1 à 9, dans la fabrication d'un médicament pour prévenir la formation de la plaque bactérienne et/ou du tartre, sur les surfaces des dents d'animaux.
